# DEMANDE DE BREVET EUROPEEN

(11) **EP 1 944 011 A1**
(43) Date de publication de la demande: **16.07.2008**
(21) Numéro de dépôt: 08300015.8
(22) Date de dépôt: 09.01.2008
(51) Int. Cl.: A61K 8/44, A61Q 5/04

(54) **Composition réductrice destinée à être mise en oeuvre dans un procédé de déformation permanente des fibres kératiniques comprenant de la cystéine et de l´acide thiolactique ou l´un de leurs sels**

(30) Priorité: 12.01.2007 FR 0752659
(71) Demandeur: L'Oréal, 75008 Paris (FR)
(72) Inventeur: Pallanchard, Marion, 92120, MONTROUGE (FR); Dutheil-Gouret, Katia, 78370, PLAISIR (FR); Sabbagh, Anne, 92500, RUEIL MALMAISON (FR)
(74) Mandataire: Catherine, Alain

(57) **Abrégé**

L'invention concerne une composition aqueuse réductrice non colorante, pour la déformation permanente des fibres kératiniques, comprenant, dans un milieu cosmétiquement acceptable :
(a) de la cystéine ou l'un de ses sels, et
(b) de l'acide thiolactique ou l'un de ses sels,

dans laquelle le rapport entre (a) le nombre de moles de cystéine ou de ses sels et (b) le nombre de moles d'acide thiolactique ou de ses sels est supérieur ou égal à 3.

La composition réductrice telle que définie ci-dessus est plus stable vis-à-vis de la formation de particules blanches, et de l'apparition d'une odeur soufrée, en comparaison des compositions réductrices connues comprenant de la cystéine.

## Description

La présente invention se rapporte à une composition réductrice destinée à être mise en oeuvre dans un procédé de déformation permanente des fibres kératiniques, en particulier les cheveux, ladite composition comprenant de la cystéine et de l'acide thiolactique ou l'un de ses sels, dans un rapport de concentration molaire spécifique.

La technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur adapté (étape de réduction) puis, après avoir rincé la chevelure ainsi traitée, généralement à l'eau, à reconstituer dans un second temps lesdites liaisons disulfures en appliquant, sur les cheveux préalablement mis sous tension (bigoudis et autres) ou mis en forme ou lissés par d'autres moyens, une composition oxydante (étape d'oxydation, dite aussi de fixation) de façon à donner finalement aux cheveux la forme recherchée. Cette technique permet ainsi de réaliser indifféremment soit l'ondulation des cheveux (procédé de permanente), soit leur défrisage ou leur décrêpage (procédé de lissage). La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que la mise en pli.

Les compositions réductrices utilisées pour la mise en oeuvre de la première étape d'une opération de permanente peuvent comprendre, à titre d'agent réducteur, de la cystéine. Or, les compositions réductrices contenant de la cystéine (L-cystéine, D-cystéine, ou D, L-cystéine) se dégradent dans le temps. La cystéine se dégrade au cours du temps, sous l'effet de réactions d'oxydations, et forme des particules blanchâtres de cystine accompagnées d'une odeur fortement soufrée.

Il existe donc un besoin de disposer d'une composition comprenant de la cystéine plus stable, en comparaison des compositions réductrices connues comprenant de la cystéine.

La demanderesse a découvert que les problèmes de l'art antérieur étaient résolus par une composition réductrice comprenant de la cystéine et de l'acide thiolactique ou l'un de ses sels, dans un rapport de concentration molaire spécifique.

L'invention a donc pour objet une composition aqueuse réductrice non colorante, pour la déformation permanente des fibres kératiniques, en particulier les cheveux, comprenant, dans un milieu cosmétiquement acceptable :
(a) de la cystéine ou l'un de ses sels, et
(b) de l'acide thiolactique ou l'un de ses sels,
dans laquelle le rapport entre (a) le nombre de moles de cystéine ou de ses sels et (b) le nombre de moles d'acide thiolactique ou de ses sels est supérieur ou égal à 3.

Par composition non colorante on entend au sens de la présente invention une composition qui ne modifie pas de manière substantielle la couleur des fibres kératiniques après application.

De manière plus précise, on considère que la composition est non colorante lorsque le paramètre L de la couleur des fibres kératiniques exprimé dans le système Lab ne diminue pas de plus de 5 unités avant et après traitement.

De préférence, les compositions de l'invention ne comprennent pas de colorants directs ou d'oxydation.

Comme cela est illustré dans les exemples, la composition réductrice telle que définie ci-dessus est plus stable que les compositions réductrices connues, notamment vis-à-vis de la formation de particules blanches de cystine, et de l'apparition d'une odeur soufrée.

Selon un mode de réalisation préféré, le rapport entre (a) le nombre de moles de cystéine ou de ses sels et (b) le nombre de moles d'acide thiolactique ou de ses sels est supérieur ou égal à 3,5, et de manière tout à fait préférée supérieur ou égal à 3,5 et inférieur ou égal à 4, de préférence, inférieur à 3,9, et plus préférentiellement inférieur à 3,8.

De préférence, la cystéine mise en oeuvre est la L-cystéine, la D-cystéine ou la L,D-cystéine. De préférence, les sels de cystéine sont des chlorhydrates ou des sulfates.

Lorsqu'un sel d'acide thiolactique est utilisé celui-ci est de préférence un sel de métal alcalin ou un sel d'ammonium.

Le pH de la composition réductrice selon l'invention est de préférence supérieur à 7, et de préférence compris entre 8 et 9,5.

Le pH de la composition réductrice selon l'invention peut être obtenu et/ou ajusté classiquement par ajout soit d'agents alcalins, tels que par exemple l'ammoniaque, la monoéthanolamine, la diéthanolamine, la triéthanolamine, l'isopropanolamine, le 2-amino 2-méthyl 1-propanol, la propanediamine-1,3, un carbonate ou bicarbonate alcalin ou d'ammonium, par exemple la carbonate et le bicarbonate de sodium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

La cystéine ou ses sels et l'acide thiolactique ou ses sels représentent généralement chacun de 0,05 à 30%, de préférence de 1 à 20%, mieux de 3 à 11% en poids par rapport au poids total de la composition réductrice.

Dans le but d'améliorer les propriétés cosmétiques des fibres capillaires ou encore d'atténuer ou d'éviter leur dégradation, la composition réductrice utilisée dans le procédé selon l'invention peut également comprendre un ou plusieurs actifs cosmétiques.

Ce ou ces actifs sont généralement choisis parmi les silicones volatiles ou non, linéaires ou cycliques, aminées ou non, les polymères cationiques, anioniques, non ioniques ou amphotères, les peptides et leurs dérivés, les hydrolysats de protéines, les cires naturelles ou synthétiques, et en particulier les alcools gras, les agents de gonflement et de pénétration ou permettant de renforcer l'efficacité du ou des réducteurs, ainsi que d'autres composés actifs tels que les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents chélatants, les agents opacifiants, les filtres solaires, les vitamines ou provitamines, les huiles minérales, végétales ou synthétiques, ainsi que les parfums et les conservateurs, et leurs mélanges.

A titre d'agents chélatants, on peut citer par exemple le sel pentasodique de l'acide diéthylène triamine pentaacétique ou pentasodium pentetate.

L'homme du métier saurait ajouter les additifs sans perturber les propriétés des compositions de l'invention.

Au sens de la présente invention, l'expression "polymère cationique" désigne tout polymère contenant des groupements cationiques et/ou des groupements ionisables en groupements cationiques.

Les polymères cationiques éventuellement présents dans la composition réductrice selon l'invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n°2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 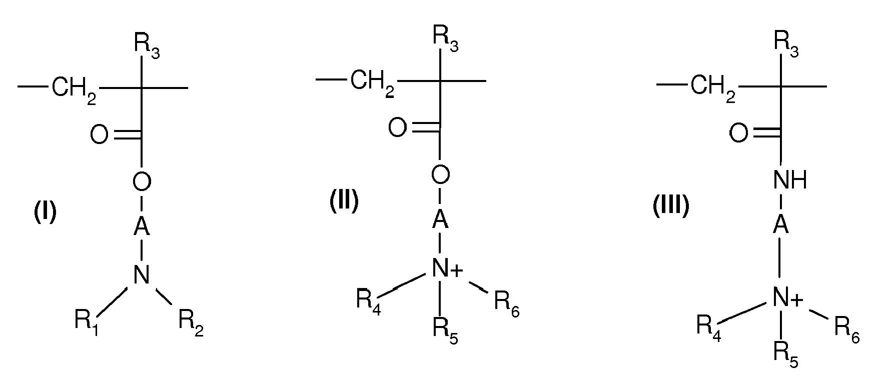 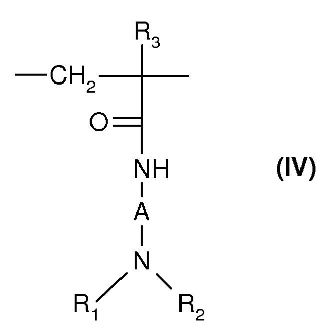 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP .
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) 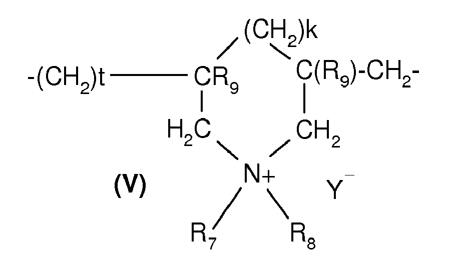 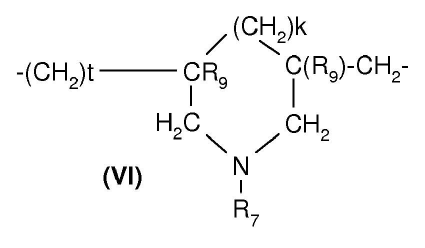 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 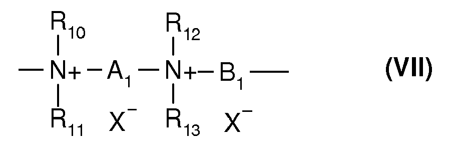 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁,
   R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
      A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un
   ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)x-CH₂-CH₂-

      -[CH₂-CH(CH₃)-O]y-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH-.
   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 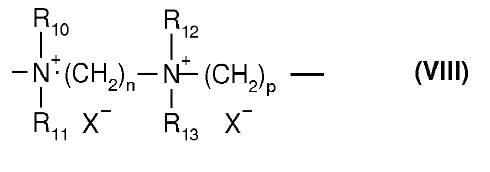 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique. On peut citer en particulier le chlorure d'hexadiméthrine (nom INCI), commercialisé par la société CHIMEX sous la référence MEXOMERE PO ;
(11) Les polyammoniums quaternaires constitués de motifs récurrents de formule (IX) : 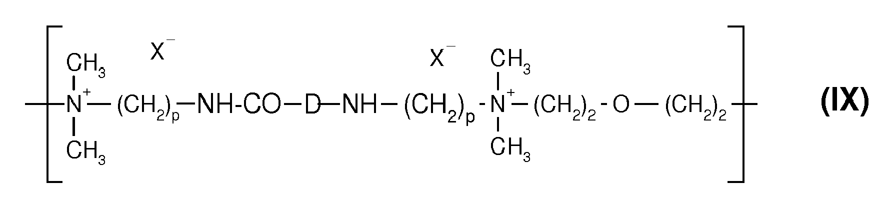 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de "SALCARE® SC 95 " et "SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre un polymère cationique de la famille (X) et par exemple le chlorure d'hexadiméthrine (nom INCI), commercialisé par la société CHIMEX sous la référence MEXOMERE PO.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Comme expliqué précédemment, le ou les actifs cosmétiques pouvant être utilisés dans la composition selon l'invention peuvent être également choisis parmi les silicones.

Les silicones éventuellement présentes dans la composition réductrice selon l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/méthylalkylsiloxane, tel que la "SILICONE VOLATILE FZ 3109" commercialisée par la société UNION CARBIDE, de structure chimique :
   avec D" : 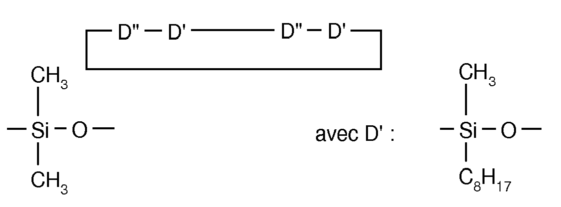 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone pouvant être présentes dans la composition réductrice selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables sont les mélanges suivants:
- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition oxydante du procédé selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition réductrice selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE, les produits 176-12096G de GENERAL ELECTRIC, les produits KF-860, 861 et 864 de SHINETSU ou les produits commercialisés sous les dénominations Q2 8220 ou DCZ-8566 et DOW CORNING 929 ou 939 ou DCZ-8299 ou Q2 7224 par la société DOW CORNING. Les groupements aminés substitués sont en particulier des groupements aminoalkyle en C₁-C₄ ; on peut citer les silicones aminées comportant des groupements alcoxy telle que la silicone BELSIL ADM LOG 1 commercialisé par la société WACKER
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

La composition réductrice objet de l'invention peut être mise en oeuvre dans des procédés de mise en forme des fibres kératiniques.

L'invention a donc également pour objet un premier procédé de déformation permanente des fibres kératiniques, en particulier des cheveux, comprenant :
- une étape d'application sur les fibres kératiniques d'une composition réductrice telle que définie ci-dessus, pour réduire les liaisons disulfure de la kératine, les fibres kératiniques étant mises sous tension mécanique avant, pendant ou après ladite application, de préférence avant, puis après un éventuel rinçage,
- une étape de fixation par oxydation, pour reformer lesdites liaisons, par application sur les fibres kératiniques d'une composition oxydante.

La composition oxydante comprend généralement au moins un agent oxydant choisi parmi le peroxyde d'hydrogène, les bromates alcalins, les polythionates, les persels, tels que les perborates, les percarbonates et les persulfates.

De préférence, l'agent oxydant est l'eau oxygénée.

Le ou les agents oxydants représentent généralement de 0,1 à 8%, de préférence de 0,2 à 5%, en poids par rapport au poids total de la composition oxydante.

De préférence, lorsque l'agent oxydant est du peroxyde d'hydrogène en solution aqueuse, la composition oxydante utilisée dans le procédé selon l'invention contient au moins un agent stabilisant de l'eau oxygénée.

On peut citer particulier les pyrophosphates des métaux alcalins ou alcalino-terreux, tel que le pyrophosphate de tétrasodium, les stannates des métaux alcalins ou alcalino-terreux, la phénacétine ou les sels d'acides et d'oxyquinoléine, comme le sulfate d'oxyquinoléine. De manière plus avantageuse encore, on utilise au moins un stannate en association ou non à au moins un pyrophosphate.

Le ou les agents stabilisants de l'eau oxygénée représentent généralement de 0,0001% à 5% en poids et de préférence de 0,01 à 2% en poids par rapport au poids total de la composition oxydante.

Dans le but d'améliorer les propriétés cosmétiques des fibres capillaires ou encore d'atténuer ou d'éviter leur dégradation, la composition oxydante utilisée dans le procédé selon l'invention peut également comprendre un ou plusieurs actifs cosmétiques, tels que ceux mentionnés précédemment au sujet de la composition réductrice.

Généralement, le pH de la composition oxydante varie de 1 à 13, de préférence de 1,5 à 8, mieux de 1,5 à 5.

Le véhicule des compositions réductrice et oxydante est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants organiques peuvent alors être présents dans des concentrations comprises entre environ 0,1 et 20% et, de préférence, entre environ 1 et 10% en poids par rapport au poids total de la composition.

Les pH de la composition oxydante utilisée dans le procédé selon l'invention peut être obtenu et/ou ajusté classiquement par ajout soit d'agents alcalins, tels que par exemple l'ammoniaque, la monoéthanolamine, la diéthanolamine, la triéthanolamine, l'isopropanolamine, le 2-amino 2-méthyl 1-propanol, la propanediamine-1,3, un carbonate ou bicarbonate alcalin ou d'ammonium, par exemple la carbonate et le bicarbonate de sodium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique, l'acide borique, l'acide citrique et l'acide phosphorique.

La composition réductrice et la composition oxydante utilisée dans le procédé selon l'invention, peuvent se présenter indépendamment sous forme de lotion, de gel, de mousse, de crème ou de pâte.

Comme expliqué précédemment, le premier procédé selon l'invention comprend une étape d'application d'une composition réductrice sur les fibres kératiniques, en particulier sur les cheveux, puis une étape d'application d'une composition oxydante.

Selon un mode de réalisation particulier de l'invention, l'étape d'application de la composition réductrice et/ou l'étape de fixation par application d'une composition oxydante s'effectue(nt) sous chauffage ou est (sont) immédiatement suivie(s) d'un chauffage.

Généralement, le procédé selon l'invention comprend, après l'étape de fixation par oxydation, une étape de rinçage des fibres kératiniques à l'eau.

Lorsque l'on souhaite réaliser une permanente, on utilise de préférence des moyens mécaniques tels que des bigoudis, la composition réductrice étant appliquée avant, pendant ou après les moyens de mise en forme des cheveux, de préférence après.

De préférence, on applique la composition réductrice sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence pendant 5 à 30 minutes.

On peut également, après application de la composition réductrice, soumettre la chevelure à un traitement thermique par chauffage à une température comprise entre 30 et 250°C pendant tout ou partie du temps de pose. Dans la pratique, cette opération peut être conduite au moyen d'un casque de coiffure, d'un sèche-cheveux, d'un fer rond ou d'un fer plat, d'un dispensateur de rayons infrarouges et d'autres appareils chauffants.

On peut notamment utiliser, à la fois comme moyen de chauffage et de mise en forme de la chevelure, un fer chauffant à une température comprise entre 60 et 220°C et de préférence entre 120 et 200°C, l'utilisation du fer chauffant se faisant entre l'étape d'application de la composition réductrice et l'étape d'application de la composition oxydante.

Le bigoudi lui-même peut-être chauffant.

On applique ensuite, sur les cheveux enroulés ou déroulés, de préférence après un rinçage, la composition oxydante permettant de reformer les liaisons disulfures de la kératine, généralement pendant un temps de pose de 2 à 15 minutes. Après avoir enlevé les rouleaux, on rince généralement abondamment la chevelure, généralement à l'eau.

Lorsque l'on souhaite effectuer le défrisage ou le décrêpage des cheveux, on applique sur les cheveux la composition réductrice, puis l'on soumet les cheveux à une déformation mécanique permettant de les fixer dans leur nouvelle forme, par une opération de lissage des cheveux avec un peigne à larges dents, avec le dos d'un peigne, à la main ou au pinceau. On observe généralement un temps de pose de 5 à 60 minutes, de préférence de 15 à 45 minutes.

Le lissage des cheveux peut également être effectué, en tout ou partie, à l'aide d'un fer chauffant entre 60 et 220°C, et de préférence entre 120 et 200°C.

Puis on applique la composition oxydante telle que définie ci-dessus, qu'on laisse généralement agir pendant environ 2 à 15 minutes, puis on rince généralement abondamment les cheveux, généralement à l'eau.

La composition réductrice selon l'invention peut également être mise en oeuvre dans un second procédé de traitement des fibres capillaires sans fixation.

L'invention a donc également pour objet un second procédé de traitement des fibres capillaires sans fixation caractérisé en ce qu'il comprend les étapes suivantes :
- application sur les fibres capillaires d'une composition réductrice telle que définie ci-dessus, les fibres kératiniques étant éventuellement mises sous tension mécanique avant, pendant ou après ladite application, de préférence avant, puis éventuellement,
- l'élévation de la température des fibres capillaires, au moyen d'un fer chauffant, à une température au moins égale à 60°C, l'élévation de la température étant effectuée avant ou après le rinçage éventuel des fibres capillaires.

Des procédés de traitement des fibres capillaires sans fixation, tels que définis ci-dessus, dans lesquels une composition réductrice conforme à l'invention peut être mise en oeuvre sont notamment décrits dans les demandes de brevet FR2868305, FR2868306 et FR2868300.

De préférence, la température des fibres capillaires est élevée jusqu'à une température comprise entre 60°C et 220°C, mieux entre 120°C et 220°C.

Selon un mode de réalisation préféré, les fibres capillaires ne sont pas rincées avant l'étape d'élévation de la température.

De préférence, la composition réductrice est appliquée sur des fibres capillaires humides et propres.

Après l'application de la composition réductrice, on peut laisser poser ladite composition, généralement pendant 5 à 60 minutes, de préférence 5 à 30 minutes, éventuellement sous casque.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre.

L'invention a également pour objet l'utilisation d'acide thiolactique ou l'un de ses sels pour stabiliser une composition réductrice non colorante, comprenant de la cystéine ou l'un de ses sels dans un milieu cosmétiquement acceptable, et dans laquelle l'acide thiolactique ou un de ses sels est ajouté à ladite composition en quantité suffisante pour que le rapport entre (a) le nombre de moles de cystéine ou de ses sels et (b) le nombre de moles d'acide thiolactique ou de ses sels soit supérieur ou égal à 3 dans la composition réductrice.

Par « stabiliser » on entend au sens de la présente invention limiter ou empêcher l'apparition de particules blanches de cystine, et/ou l'apparition d'une odeur soufrée, au sein d'une composition réductrice par rapport à une composition réductrice témoin comprenant de la cystéine et éventuellement de l'acide thiolactique ou un de ses sels, dans un rapport de concentrations molaires différent de celui défini ci-dessus.

Cette amélioration de stabilité peut être mise en évidence par des tests accélérés après stockage à 45°C.

L'invention a également pour objet un kit comprenant :
- au moins un premier compartiment renfermant une composition comportant de la cystéine ou l'un de ses sels et de l'acide thiolactique ou l'un de ses sels de telle sorte que le rapport entre le nombre de moles de cystéine ou l'un de ses sels et le nombre de moles d'acide thiolactique ou l'un de ses sels soit supérieur ou égal à 3, et
- au moins un second compartiment renfermant une composition comprenant au moins un agent oxydant.

L'invention est en outre illustrée par les exemples qui suivent.

### Exemple 1

Des exemples de formulations de compositions réductrices (1) et (2) et oxydantes (3) selon l'invention, pour la mise en oeuvre d'un procédé de déformation permanente des cheveux sont détaillées ci-dessous.

### Composition réductrice (1) : pH=9,3

| | |
|---|---|
| L-cystéine | 4% |
| Monoethanolamine | 2,5% |
| Pentasodium pentetate 40% MA* | 0,4% |
| MEXOMERE PO 60% MA | 2,5% |
| Pyrrolidone carboxylate de sodium 50% MA | 3% |
| Thiolactate d'ammonium en solution aqueuse à | 1,8% |
| 58% | |
| Eau déminéralisée | QS 100% |
| Rapport molaire cystéine/thiolactate d'ammonium | 3,86 |

### Composition réductrice (2) : pH=8,8

| | |
|---|---|
| L-cystéine | 4% |
| Monoethanolamine | 2,25% |
| bicarbonate d'ammonium | 2,7% |
| Pentasodium pentetate 40% MA* | 0,4% |
| MEXOMERE PO 60% MA | 2,5% |
| Pyrrolidone carboxylate de sodium 50% MA | 3% |
| Thiolactate d'ammonium en solution aqueuse à | 1,8% |
| 58% | |
| Eau déminéralisée | QS 100% |
| Rapport molaire cystéine/thiolactate d'ammonium | 3,86 |

### Composition oxydante (3) : pH=3

| | |
|---|---|
| Peroxyde d'hydrogène | 4,8% |
| Stabilisants | 0,03% |
| Acide citrique | 0,1% |
| MERQUAT 100 | 1,25% |
| Oxyde de lauramine | 2,15% |
| Eau déminéralisée | QS 100% |

### Exemple 2

Une étude de stabilité des compositions 1 et 2 selon l'invention a été réalisée, et comparée à la stabilité de compositions connues. Les résultats figurent ci-dessous :

### Symboles utilisés :

(-)→ RAS (solution limpide)
(+)→ quelques particules
(++)→ particules

### Formulation à pH=9,3 :

| | **Exemple comparatif** | **Composition (1) selon invention** |
|---|---|---|
| L-cystéine | 4% | |
| Monoethanolamine | 2.5% | |
| Pentasodium pentetate 40% MA* | 0.4% | |
| MEXOMERE PO 60% MA | 2.5% | |
| Pyrrolidone carboxylate de sodium 50% MA | 3% | |
| Thiolactate d'ammonium en solution aqueuse à 58% | - | 1.8% |
| Eau déminéralisée | QS 100% | |

| **Résultats T₀** | | |
|---|---|---|
| Aspect | (-) | (-) |

| **Résultats après 2 mois à 45 °C** | | |
|---|---|---|
| Aspect | (+) | (-) |

| | | |
|---|---|---|
| * Acide diéthylène triamine pentacétique, sel pentasodique en solution aqueuse à 40%. | | |

Comme on peut l'observer dans le tableau ci-dessus, la composition réductrice (1) conforme à l'invention reste limpide après 2 mois de stockage à 45°C, contrairement à une formulation ne comprenant pas de thiolactate d'ammonium.

### Formulation à pH=8,8 :

| | **Exemple comparatif** | **Composition (2) selon invention** |
|---|---|---|
| L-cystéi ne | 4% | |
| Monoethanolamine | 2.25% | |
| Ammonium bicarbonate | 2.7% | |
| Pentasodium pentetate 40% MA* | 0.4% | |
| MEXOMERE PO 60% MA | 2.5% | |
| Pyrrolidone carboxylate de sodium 50% MA | 3% | |
| Thiolactate d'ammonium en solution aqueuse à 58% | - | 1.8% |
| Eau déminéralisée | QS 100% | |

| **Résultats T₀** | | |
|---|---|---|
| Aspect | (-) | (-) |

| **Résultats après 2 mois à 45 °C** | | |
|---|---|---|
| Aspect | (++) | (+) |

| | | |
|---|---|---|
| * Acide diéthylène triamine pentacétique, sel pentasodique en solution aqueuse à 40%. | | |

Comme on peut l'observer dans le tableau ci-dessus, la composition réductrice (2) conforme à l'invention présente moins de particules blanches après 2 mois de stockage à 45°C, qu'une formulation ne comprenant pas de thiolactate d'ammonium.

### Exemple 3 : procédés

### 1- Procédé de permanente sur cheveux naturels

La chevelure d'un modèle aux cheveux naturels est lavée au moyen d'un shampoing, puis essorée. La chevelure complète est enroulée sur bigoudis.

La composition réductrice 1 est appliquée, un bonnet est placé sur la tête.

Une pause de 20 minutes est observée, suivie d'un rinçage à l'eau.

La composition oxydante 3 est alors appliquée et une pause de 7 minutes est observée.

Les bigoudis sont alors enlevés, et la chevelure est rincée.

Après séchage, on obtient des boucles régulières et un toucher de cheveux très naturel.

### 2- Procédé de permanente sur cheveux colorés

La chevelure d'un modèle aux cheveux colorés est lavée au moyen d'un shampoing, puis essorée. La chevelure complète est enroulée sur bigoudis.

La composition réductrice 2 est appliquée, un bonnet est placé sur la tête.

Une pause de 20 minutes est observée, suivie d'un rinçage à l'eau.

La composition oxydante 3 est alors appliquée et une pause de 7 minutes est observée.

Les bigoudis sont alors enlevés, et la chevelure est rincée.

Après séchage, on obtient des boucles régulières et la couleur de la chevelure a été conservée à l'identique.

### 3- Procédé sans fixation sur cheveux naturels

La chevelure d'un modèle aux cheveux naturels est lavée au moyen d'un shampoing, puis essorée.

La composition réductrice 1 est appliquée sur l'ensemble de la chevelure.

Une pause de 15 minutes sous casque chauffant est observée, suivie d'un rinçage à l'eau.

La chevelure est séchée avec un sèche-cheveux jusqu'à atteindre environ 20% d'humidité résiduelle.

Un fer plat est alors passé sur la chevelure, en prenant des mèches fines et en effectuant 3 passages de fer sur chaque mèche.

On obtient une chevelure lisse et un toucher de cheveux très naturel.

## Revendications

1. Composition aqueuse réductrice non colorante, pour la déformation permanente des fibres kératiniques, comprenant, dans un milieu cosmétiquement acceptable :
(a) de la cystéine ou l'un de ses sels, et
(b) de l'acide thiolactique ou l'un de ses sels,
dans laquelle le rapport entre (a) le nombre de moles de cystéine ou de ses sels et (b) le nombre de moles d'acide thiolactique ou de ses sels est supérieur ou égal à 3.

2. Composition selon la revendication 1 **caractérisée en ce qu'**elle présente un pH supérieur à 7, et de préférence compris entre 8 et 9,5.

3. Composition selon la revendication 1 ou 2, **caractérisé en ce que** la cystéine est la L-cystéine, la D-cystéine ou la L,D-cystéine.

4. Composition selon l'une quelconque des revendications précédentes, **caractérisé en ce que** le sel de cystéine est un chlorhydrate ou un sulfate.

5. Composition selon l'une quelconque des revendications précédentes, **caractérisé en ce que** le sel d'acide thiolactique est un sel de métal alcalin ou un sel d'ammonium.

6. Composition selon l'une quelconque des revendications précédentes **caractérisée en ce que** la cystéine ou ses sels et l'acide thiolactique ou ses sels représentent chacun de 0,05 à 30%, de préférence de 1 à 20%, mieux de 3 à 11% en poids par rapport au poids total de la composition réductrice.

7. Composition selon l'une quelconque des revendications précédentes **caractérisé en ce qu'**elle comprend un ou plusieurs solvants choisis parmi l'eau, les alcools en C₁-C₆, de préférence les alcanols tels que l'éthanol, le propanol et l'isopropanol, les polyols tels que le glycérol, le propylène glycol et le pentanediol, l'alcool benzylique, les éthers de polyols, les esters en C₂-C₆, la N-méthylpyrrolidone (NMP), et les cétones en C₃-C₆.

8. Composition selon l'une quelconque des revendications précédentes **caractérisé en ce qu'**elle comprend un ou plusieurs additifs cosmétiques choisis parmi les silicones volatiles ou non, linéaires ou cycliques, les polymères cationiques, non-ioniques, anioniques ou amphotères, les peptides et leurs dérivés, les hydrolysats de protéines, les cires, les agents de gonflement et de pénétration ou permettant de renforcer l'efficacité du réducteur tels que le mélange SiO₂/polydiméthylsiloxane, le diméthylisosorbitol, l'urée et ses dérivés, la thiamorpholinone, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les filtres solaires, les vitamines ou provitamines, les acides gras, les alcools gras, les huiles minérales, végétales ou synthétiques, ainsi que les parfums et les conservateurs, et leurs mélanges.

9. Composition selon l'une quelconque des revendications précédentes **caractérisé en ce que** le polymère cationique est un chlorure d'hexadiméthrine.

10. Composition selon l'une quelconque des revendications précédentes **caractérisé en ce qu'**elle se présente sous la forme d'une lotion, épaissie ou non, d'une crème ou d'un gel ou d'une mousse.

11. Procédé de déformation permanente des fibres kératiniques, en particulier des cheveux, comprenant :
- une étape d'application sur les fibres kératiniques d'une composition réductrice telle que définie selon l'une quelconque des revendications 1 à 10, pour réduire les liaisons disulfure de la kératine, les fibres kératiniques étant mises sous tension mécanique avant, pendant ou après ladite application, de préférence avant, puis après un éventuel rinçage,
- une étape de fixation par oxydation, pour reformer lesdites liaisons, par application sur les fibres kératiniques d'une composition oxydante.

12. Procédé selon la revendication 11 **caractérisé en ce que** la composition oxydante comprend au moins un agent oxydant choisi parmi le peroxyde d'hydrogène, les bromates alcalins, les polythionates, les persels, tels que les perborates, les percarbonates et les persulfates.

13. Procédé selon la revendication 12 **caractérisé en ce que** le ou les agents oxydants représentent généralement de 0,1 à 8%, de préférence de 0,2 à 5%, en poids par rapport au poids total de la composition oxydante.

14. Procédé selon l'une quelconque des revendications 11 à 13 **caractérisé en ce que** le pH de la composition oxydante varie de 1 à 13, de préférence de 1,5 à 8, mieux de 1,5 à 5.

15. Procédé selon l'une quelconque des revendications 11 à 14 **caractérisé en ce que** l'étape d'application de la composition réductrice et/ou l'étape de fixation s'effectue(nt) sous chauffage ou est (sont) immédiatement suivie(s) d'un chauffage.

16. Procédé de traitement des fibres capillaires sans fixation **caractérisé en ce qu'**il comprend les étapes suivantes :
- application sur les fibres capillaires d'une composition réductrice telle que définie selon l'une quelconque des revendications 1 à 10, les fibres kératiniques étant éventuellement mises sous tension mécanique avant, pendant ou après ladite application, de préférence avant, puis éventuellement,
- l'élévation de la température des fibres capillaires, au moyen d'un fer chauffant, à une température au moins égale à 60°C, l'élévation de la température étant effectuée avant ou après le rinçage éventuel des fibres capillaires.

17. Procédé selon la revendication 16 **caractérisé en ce que** la température est élevée jusqu'à une température comprise entre 60°C et 220°C, de préférence entre 120°C et 220°C.

18. Procédé selon la revendication 11 ou 17, **caractérisé en ce que** la composition réductrice est appliquée sur des fibres capillaires humides et propres.

19. Procédé selon l'une quelconque des revendications 10 à 18, **caractérisé en ce que**, après l'application de la composition réductrice, on laisse poser ladite composition réductrice.

20. Procédé selon l'une quelconque des revendications 15 à 19, **caractérisé en ce que** les fibres capillaires ne sont pas rincées avant l'étape d'élévation de la température.

21. Utilisation d'acide thiolactique ou l'un de ses sels pour stabiliser une composition réductrice non colorante, comprenant de la cystéine ou l'un de ses sels dans un milieu cosmétiquement acceptable, et dans laquelle l'acide thiolactique ou le sel d'acide thiolactique est ajouté à ladite composition en quantité suffisante pour que le rapport entre (a) le nombre de moles de cystéine ou de ses sels et (b) le nombre de moles d'acide thiolactique ou de ses sels soit supérieur ou égal à 3 dans la composition réductrice.

22. kit comprenant :
- au moins un premier compartiment renfermant une composition comportant de la cystéine ou l'un de ses sels et de l'acide thiolactique ou l'un de ses sels, de telle sorte que le rapport entre le nombre de moles de cystéine ou l'un de ses sels et le nombre de moles d'acide thiolactique ou l'un de ses sels soit supérieur ou égal à 3, et
- au moins un second compartiment renfermant une composition comprenant au moins un agent oxydant.
